# EUROPEAN PATENT APPLICATION

(11) **EP 3 804 604 A1**
(43) Date of publication of application: **14.04.2021**
(21) Application number: 19811542.0
(22) Date of filing: 28.02.2019
(51) Int. Cl.: A61B 1/045, A61B 1/00

(54) **CAMERA APPARATUS, IMAGE PROCESSING METHOD, AND CAMERA SYSTEM**

(30) Priority: 31.05.2018 JP 2018105397
(71) Applicant: Panasonic i-PRO Sensing Solutions Co., Ltd., Fukuoka City 812-8531 (JP)
(72) Inventor: HIRANO, Tetsushi, Fukuaka 812-8531 (JP); TABEI, Kenji, Osaka 571-8501 Japan (JP); SAITO, Tomoyuki, Osaka 571-8501 Japan (JP); KINIWA, Yuji, Fukuoka 812-8531 Japan (JP)
(74) Representative: Eisenführ Speiser
(86) International application number: PCT/JP2019/007801
(87) International publication number: WO 2019/230095

(57) **Abstract**

This camera apparatus is provided with: a camera head that is able to perform imaging based on visible light having entered a medical optical device from a target portion of a subject to whom a florescence agent has been administered in advance and imaging based on fluorescence having entered the medical optical device from the target portion; and an image processing unit that, after amplifying the intensity of a fluorescence image inputted from the camera head and increasing the contrast between a black part and a white part in the fluorescence image, executes nonlinear conversion processing for the amplified fluorescence image, superimposes the fluorescence image having undergone the nonlinear conversion processing onto a visible image obtained by the imaging based on visible light, and generates a superimposed image for being outputted to an output unit.

## Description

### TECHNICAL FIELD

The present disclosure relates to a camera device, an image processing method, and a camera system for processing an image that has been taken during a medical act, for example.

### BACKGROUND ART

In microscope surgeries that are performed while a minute surgery target part (e.g., a diseased portion of a subject body) is observed using a surgical microscope and endoscope surgeries that are performed while a surgery target part in a subject body is observed using an endoscope, an observation image (e.g., an ordinary visible image or a fluorescence image with excitation by IR excitation light) of the surgery target part is taken and displayed on a monitor. Displaying an observation image on the monitor allows a doctor or the like to check a state of the surgery target part in detail and recognize the state of the surgery target part in real time.

PTL 1 discloses an endoscope device in which brightness of a lighting control target is calculated by multiplying brightness levels of a fluorescence image and a reference light image calculated by a fluorescence image brightness calculation circuit and a reference light image brightness calculation circuit are multiplied by a first coefficient and a second coefficient stored in a coefficient storage memory, respectively, and adding resulting products and a gain to be set as a lighting control target value is calculated through a gain calculation circuit to make adjustments.

### CITATION LIST

### PATENT LITERATURE

[PTL 1]: JP-A-2015-054038

### SUMMARY OF INVENTION

### TECHNICAL PROBLEM

In medical surgeries such as microscope surgeries and endoscope surgeries as described above, to make it possible to check a clear state of a target part to be subjected to the surgery, a treatment, or the like (e.g., a part of a subject body to which a fluorescent chemical was administered by, for example, injection before the surgery), it is desired to receive a high-visibility output video from a camera system for taking an observation image and to display it. The visibility of the output video displayed on a monitor is important for a doctor or the like to recognize, in detail, a state of the target part (e.g., a diseased portion of the subject body). It is therefore desired that, for example, a fluorescence image taken using fluorescence generated by excitation of a fluorescent chemical by IR excitation light be high in image quality.

However, fluorescence generated by excitation of a fluorescent chemical such as ICG (indocyanine green) by IR excitation light is low in light intensity as compared with the IR excitation light. This results in a problem that when a fluorescence portion in a fluorescence image is superimposed on a visible image to make a state of a diseased portion easier to see, a boundary, for example, between the visible image and the fluorescence portion is difficult to determine and the fluorescence portion is difficult to see. This makes a doctor or the like to recognize the diseased portion in detail and hence causes inconvenience in a surgery, a treatment, or the like. Furthermore, since the skin thickness etc. vary from one subject body (i.e., patient) to another, the fact that how a fluorescence image looks does not necessarily the same is another factor in causing inconvenience. No technical measure against the above problems is disclosed in Patent document 1, and it can be said that how to solve the above-described problems is not considered as yet in the art.

The concept of the present disclosure has been conceived in view of the above circumstances in the art, and an object of the disclosure is therefore to provide a camera device, an image processing method, and a camera system that suppress lowering of the image quality of a fluorescence image and make a fluorescence portion in a fluorescence image easier to see when the fluorescence image is superimposed on an ordinary visible image and thereby assist output of a video taken that allows a user such as a doctor to check a clear state of a target part of a subject body.

### SOLUTION TO PROBLEM

The disclosure provides a camera device including a camera head which can perform both of imaging on the basis of visible light shining on a medical optical device from a target part of a subject body to which a fluorescent chemical was administered in advance and imaging on the basis of fluorescence shining on the medical optical device from the target part; and an image processing unit which performs nonlinear conversion processing on an amplified fluorescence image after the intensity of a fluorescence image that is input from the camera head is amplified and a black portion and a white portion of the fluorescence image are emphasized, and generates a superimposed image to be output to an output unit by superimposing a fluorescence image as subjected to the nonlinear conversion processing on a visible image obtained by imaging on the basis of visible light.

Furthermore, the disclosure provides an image processing method employed in a camera device including a camera head and an image processing unit. The image processing method includes the steps of causing the camera head to perform each of imaging on the basis of visible light shining on a medical optical device from a target part of a subject body to which a fluorescent chemical was administered in advance and imaging on the basis of fluorescence shining on the medical optical device from the target part; and causing the image processing unit to perform nonlinear conversion processing on an amplified fluorescence image after the intensity of a fluorescence image that is input from the camera head is amplified and a black portion and a white portion of the fluorescence image are emphasized, and generates a superimposed image to be output to an output unit by superimposing a fluorescence image as subjected to the nonlinear conversion processing on a visible image obtained by the imaging on the basis of visible light.

Still further, the disclosure provides a camera system including a camera device and an output unit. The camera device performs each of imaging on the basis of visible light shining on a medical optical device from a target part of a subject body to which a fluorescent chemical was administered in advance and imaging on the basis of fluorescence shining on the medical optical device from the target part; and performs nonlinear conversion processing on an amplified fluorescence image after the intensity of a fluorescence image that is obtained by the imaging on the basis of the fluorescent is amplified and a black portion and a white portion of the fluorescence image are emphasized, and generates a superimposed image to be output to an output unit by superimposing a fluorescence image as subjected to the nonlinear conversion processing on a visible image obtained by the imaging on the basis of visible light; and the output unit outputs the superimposed image generated by the camera device.

### ADVANTAGEOUS EFFECTS OF INVENTION

The present disclosure makes it possible to suppress lowering of the image quality of a fluorescence image and make a fluorescence portion in a fluorescence image easier to see when the fluorescence image is superimposed on an ordinary visible image and thereby assist output of a video taken that allows a user such as a doctor to check a clear state of a target part of a subject body.

### BRIEF DESCRIPTION OF DRAWINGS

[FIG. 1] FIG. 1 is a diagram showing an example system configuration in which a medical camera system including a camera device according to a first or second embodiment is applied to a surgical microscope system.
[FIG. 2] FIG. 2 is a view showing an example appearance of the surgical microscope system.
[FIG. 3] FIG. 3 is a block diagram showing an example hardware configuration of the camera device according to the first embodiment.
[FIG. 4] FIG. 4 is a block diagram showing an example detailed hardware configuration of a visible video/IR video superimposing unit.
[FIG. 5A] FIG. 5A is an explanatory diagram of threshold processing.
[FIG. 5B] FIG. 5B is an explanatory diagram illustrating a first example (binarization) of nonlinear conversion processing.
[FIG. 5C] FIG. 5C is an explanatory diagram illustrating a second example (conversion into an N-ary value) of the nonlinear conversion processing.
[FIG. 6] FIG. 6 is a flowchart showing an example operation procedure of the camera device according to the first embodiment.
[FIG. 7] FIG. 7 is example schematic views showing how an IR video is varied by the threshold processing and the nonlinear conversion processing.
[FIG. 8] FIG. 8 shows an example superimposed video in which an IR video is superimposed on a visible video.
[FIG. 9] FIG. 9 is a block diagram showing an example hardware configuration of a camera device according to a second embodiment.
[FIG. 10] FIG. 10 is views showing display examples for comparison between a visible video and a superimposed video.
[FIG. 11] FIG. 11 is views showing display examples for comparison between an IR video and the superimposed video.
[FIG. 12] FIG. 12 is views showing display examples for comparison between the visible video, the IR video, and the superimposed video.
[FIG. 13] FIG. 13 is a system configuration diagram in which a medical camera system including the camera device according to the first or second embodiment is applied to a surgical endoscope system.
[FIG. 14] FIG. 14 is a view showing an example appearance of the surgical endoscope system.

### DESCRIPTION OF EMBODIMENTS

Each embodiment in which a camera device, an image processing method, and a camera system according to the present disclosure disclosed in a specific manner will be described in detail by referring to the drawings when necessary. However, unnecessarily detailed descriptions may be avoided. For example, detailed descriptions of already well-known items and duplicated descriptions of constituent elements having substantially the same ones already described may be omitted. This is to prevent the following description from becoming unnecessarily redundant and thereby facilitate understanding of those skilled in the art. The following description and the accompanying drawings are provided to allow those skilled in the art to understand the disclosure thoroughly and are not intended to restrict the subject matter set forth in the claims.

In each embodiment described below, a medical camera system that is used for a medical surgery such as a microscope surgery or an endoscope surgery will be described as an example camera system including the camera device according to the disclosure. However, the camera system is not limited to a medical camera system as described in this example.

### (Embodiment 1)

In the first embodiment, the camera device performs each of imaging that uses visible light coming from an observation target part (e.g., a diseased portion as a target part of a surgery) of a subject body (e.g., patient) to which a fluorescent chemical such as ICG (indocyanine green) was administered in advance and shining on a medical optical device and imaging that uses fluorescence coming from the observation target part and shining on the medical optical device. For example, the medical optical device is a surgical microscope or a surgical endoscope. The camera device performs image processing including at least nonlinear conversion processing on a fluorescence image obtained by imaging on the basis of fluorescence and generates a superimposed image to be output to an output unit by superimposing a fluorescence image obtained by the image processing on a visible image obtained by imaging on the basis of visible light.

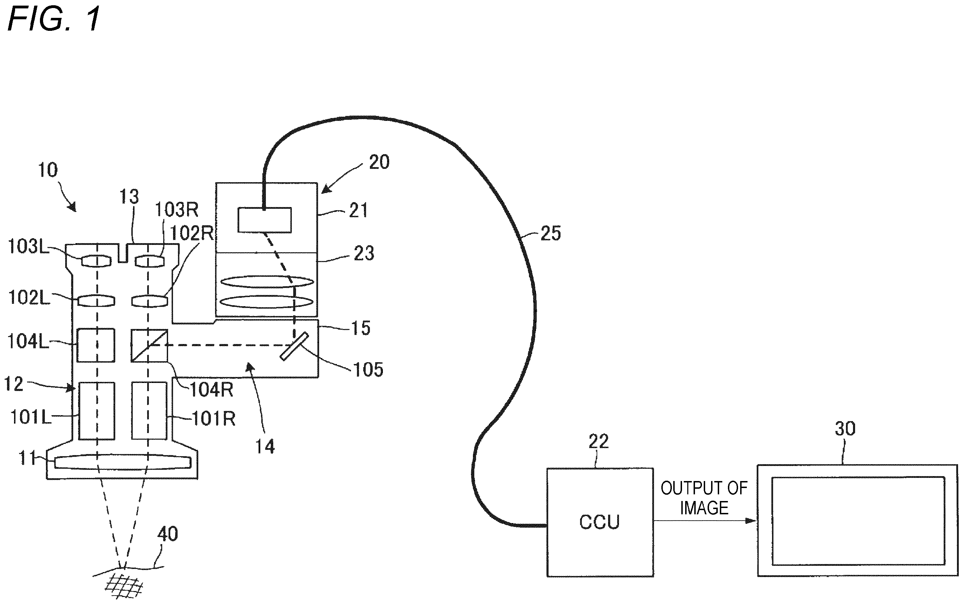

FIG. 1 is a system configuration diagram showing an example configuration in which a medical camera system including a camera device 20 according to a first or second embodiment is applied to a surgical microscope system. The surgical microscope system is configured so as to include a surgical microscope 10 as an example medical optical device, the camera device 20, and an output unit 30. The camera device 20 has a camera head 21 which takes an observation video of an observation target part on the basis of light received from the surgical microscope 10 by focusing, on an imaging unit 24, light incident on an imaging optical system 23. The camera device 20 has a CCU (camera control unit) 22 that performs image processing on each of observation image frames constituting an observation video taken by the camera head 21. In the camera device 20, the camera head 21 and the CCU 22 are connected to each other by a signal cable 25. The camera head 21 is attached and connected to a camera mounting unit 15 of the surgical microscope 10. The output unit 30 (e.g., a display device of a monitor or the like) for displaying an observation video that is a result of image processing by the CCU 22 is connected to an output terminal of the CCU 22.

The surgical microscope 10, which is a binocular microscope, for example, is configured so as to have an objective lens 11, an observation optical system 12 having units that are provided so as to correspond to the left and right eyes of an observer such as a doctor, an eyepiece unit 13, a camera imaging optical system 14, and the camera mounting unit 15. In the observation optical system 12, zoom optical systems 101L and 101R, imaging lenses 102L, and 102R, and eyepiece lenses 103 and 103R are disposed so as to correspond to the left and right eyes of an observer. The zoom optical systems 101L and 101R, the imaging lenses 102L, and 102R, and the eyepiece lenses 103 and 103R are disposed so as to be symmetrical with respect to the optical axis of the objective lens 11. Light beams carrying left and light observation images having a parallax are obtained in such a manner that light beams produced from a subject body 40 shine on the objective lens 11 and then pass through the zoom optical systems 101L and 101R, the imaging lenses 102L and 102R, the eyepiece lenses 103 and 103R, an optical system 104L, and a beam splitter 104R, and are guided to the eyepiece unit 13. An observer can see, stereoscopically, a state of an observation part of the subject body 40 by looking through the eyepiece unit 13 with his or her both eyes.

The above-mentioned light produced from the subject body 40 is reflection light that is produced in such a manner that white light (e.g., ordinary RGB visible light) emitted from a light source device 31 (described later) is applied to the observation target part of the subject body 40 to whom a fluorescent chemical such as ICG (mentioned above) was administered in advance by, for example, injection and reflected from the observation target part or fluorescence that is emitted as a result of excitation of the fluorescent chemical by IR excitation light emitted from the light source device 31. In the surgical microscope 10, to prevent lowering of image quality of a fluorescence image taken by fluorescence imaging, it is preferable that a band cut filter (BCF) for interrupting the IR excitation light be formed between the objective lens 11 and each of the zoom optical systems 101L and 101R.

In a microscope surgery or an endoscope surgery, when ICG (indocyanine green) which is a fluorescent chemical is administered to the body (an observation part) of a subject body 40 in advance of illumination with IR excitation light to allow a doctor or the like to recognize a state of a lymph node of the observation part (e.g., a diseased portion of a subject body 40), ICG (indocyanine green) gathers in the diseased portion which is a subject. When excited by IR excitation light, ICG (indocyanine green) emits fluorescence on the longer wavelength side (e.g., 860 nm). The wavelength of IR excitation light is 780 nm or 808 nm, for example. Shooting using light (i.e., fluorescence) generated by this fluorescence emission makes it possible to recognize a state of the diseased portion in detail.

The camera imaging optical system 14 has the optical system 104L, the beam splitter 104R, and a mirror 105R. The camera imaging optical system 14 separates part of light passing through the observation optical system 12 by deflecting it by the beam splitter 104R and guides it to the camera mounting unit 15 by reflecting it by the mirror 105R.

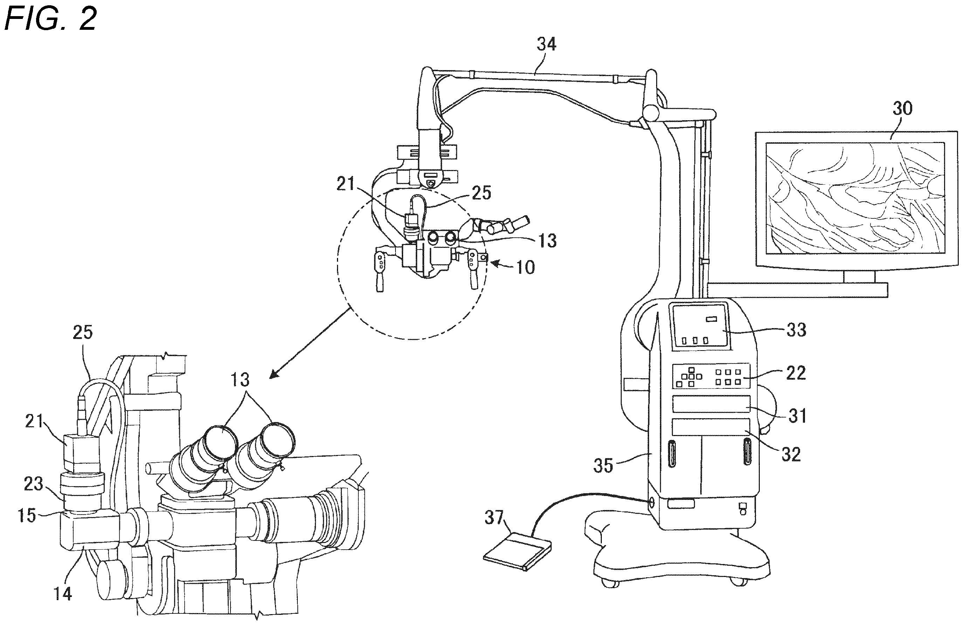

FIG. 2 is a view showing an example appearance of the surgical microscope system. In the surgical microscope 10, the eyepiece unit 13 is provided at the top of the microscope main body and the body of the camera imaging optical system 14 extends sideways from a base portion of the eyepiece unit 13 and is provided with the camera mounting unit 15. The camera mounting unit 15 has a top opening and is thus configured so that the imaging optical system 23 of the camera head 21 can be attached to it. The imaging optical system 23 can be replaced by detaching it from the main body of the camera head 21 and attaching a new one and hence imaging optical systems having different optical characteristics can be used for respective uses. For example, the camera head 21 is constituted by a four-plate imaging unit having a spectral prism for separating light carrying a subject image into light beams in respective wavelength ranges of R, G, and B (red, green, and blue) and IR (infrared radiation) and four image sensors for imaging subject images carried by light beams in the wavelength ranges of R, G, and B and IR, respectively. Alternatively, a single-plate imaging unit having one image sensor in which R, G, and B and IR pixels are arranged may be used as the imaging unit 24. As a further alternative, a two-plate imaging unit having a prism for separating incident light into visible light and IR light (e.g., fluorescence) and two image sensors, that is, an image sensor for imaging a visible light image and an image sensor for imaging an IR light (e.g., fluorescence) image, may be used as the imaging unit 24.

The surgical microscope system is configured so as to include a light source device 31 for illuminating a target part, a recorder 32 for recording an observation image taken by the camera device 20, a manipulation unit 33 for manipulating the surgical microscope system, and a foot switch 37 that allows an observer to make a manipulation input by his or her foot. The manipulation unit 33, the CCU 22, the light source device 31, and the recorder 32 are housed in a control unit body 35. The output unit 30 (e.g., a display such as a liquid crystal display) is disposed in the vicinity of the control unit body 35. The surgical microscope 10 is attached to a support arm 34 capable of displacement and is connected to the control unit body 35 by a support arm 34.

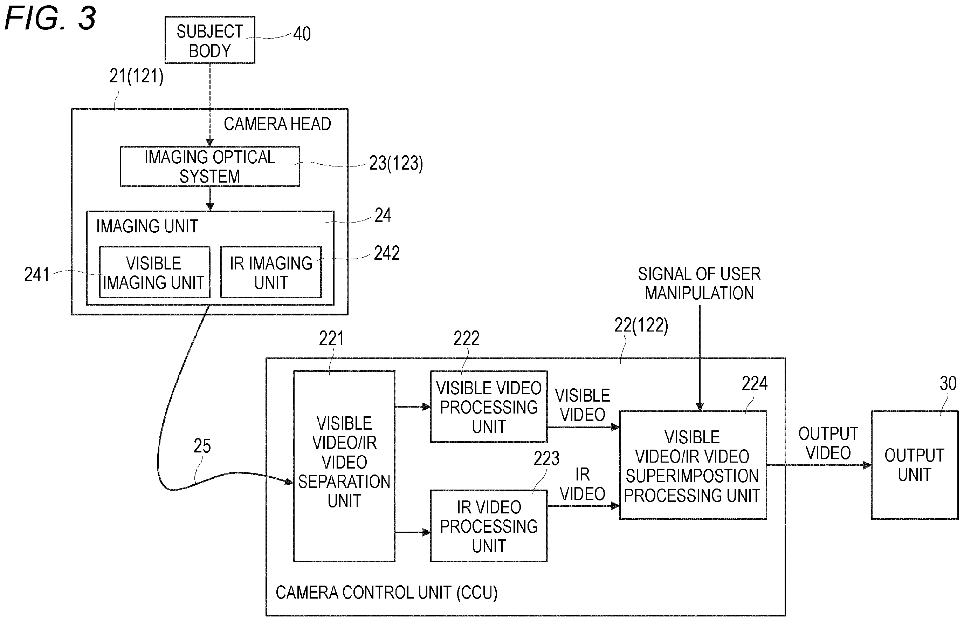

FIG. 3 is a block diagram showing an example hardware configuration of the camera device 20 according to the first embodiment. The camera device 20 shown in FIG. 3 is configured so as to include the camera head 21 or 121 and the CCU 22 or 122. The camera head 21 or 121 and the CCU 22 or 122 are connected to each other by the signal cable 25.

The camera head 21 has the imaging optical system 23 or 123 and the imaging unit 24. The camera head 21 is attached to the camera mounting unit 15 of the surgical microscope 10 at the time of a microscope surgery, for example. In the camera head 21, light coming from a subject body 40 passes through the imaging optical system 23 or 123 and image-formed on the imaging surfaces of the image sensors that are held by a visible imaging unit 241 and an IR imaging unit 242 of the imaging unit 24, whereby R, G, and B subject images and an IR subject image are taken.

The imaging optical system 23 or 123 has one or plural lenses and a spectral prism for separating light coming from a subject body 40 into light beams in R, G, and B and IR wavelength ranges.

The imaging unit 24 has the visible imaging unit 241 and the IR imaging unit 242.

The visible imaging unit 241 is configured using a three-plate image sensor that is disposed so as to be able to image, for example, images carried by light beams in the R, G, and B wavelength ranges or a single-plate image sensor in which R, G, and B pixels are arranged, and generates a visible light observation video (hereinafter also referred to as a "visible video" or a "visible image") on the basis of light beams in the R, G, and B wavelength ranges that have passed through the imaging optical system 23 or 123.

The IR imaging unit 242 is configured using a single-plate image sensor that is disposed so as to be able to image, for example, an image carried by G (green) or R (red) light, and generates a fluorescence observation video (hereinafter also referred to as an "IR video" or an "IR image") on the basis of light in the IR wavelength range (i.e., fluorescence) that has passed through the imaging optical system 23 or 123.

The (or each) image sensor is constituted by a solid-state imaging device such as a CCD (charge-coupled device) or a CMOS (complementary metal-oxide-semiconductor) sensor. A signal of an observation video of an observation target part (i.e., diseased portion) of a subject body 40 taken by the camera head 21 is transmitted by the signal cable 25 and input to the CCU 22.

The CCU 22 or 122, which is an example of a term "image processing device," is configured using a visible video/IR video separation unit 221, a visible video processing unit 222, an IR video processing unit 223, and a visible video/IR video superimposition processing unit 224. Each unit of the CCU 22 or 122 is configured using a CPU (central processing unit), a DSP (digital signal processor), or an FPGA (field programmable fate array) and its circuit configuration and manners of operation can be set or altered by a program.

The CCU 22 or 122 receives a signal of an observation video (visible video and IR video) taken by the camera head 21, and performs prescribed image processing for visible video on the visible video(s) and performs prescribed image processing for IR video on the IR video. The CCU 22 or 122 performs prescribed kinds of image processing for improving the image quality of IR video on the IR video, generates a superimposed video by superimposes an IR video thus image-processed on a resulting visible video, and outputs the superimposed video to an output unit 30.

The visible video/IR video separation unit 221 separates the observation video signal transmitted from the camera head 21 by the signal cable 25 into a visible video signal and an IR video signal and sends the visible video signal and the IR video signal to the visible video processing unit 222 and the IR video processing unit 223, respectively. For example, where the imaging unit 24 of the camera head 21 takes visible videos and IR videos periodically in a time-divisional manner, a visible video taken is input in a first prescribed period and an IR video taken is input is input in the next prescribed period. In this case, since the visible videos are already separated from the IR videos when they are input to the visible video/IR video separation unit 221, the visible video/IR video separation unit 221 sends only the input visible video to the visible video processing unit 222 in the first prescribed period and sends only the input IR video to the IR video processing unit 223 in the next prescribed period.

For example, provided with the visible imaging unit 241 and the IR imaging unit 242, the imaging unit 24 of the camera head 21 can take a visible video and an IR video at the same time. In this case, since, for example, a visible video and an IR video are input to the visible video/IR video separation unit 221 alternately, only the visible video is sent to the visible video processing unit 222 and only the IR video is sent to the IR video processing unit 223 by discriminating and separating the visible video and the IR video by, for example, referring to header regions. A visible video and an IR video may be input to the visible video/IR video separation unit 221 at the same time. In this case, the signal cable 25 (see FIG. 3) is formed so as to include both of a signal line for visible video and a signal line for IR video.

The visible video processing unit 222 performs ordinary image processing (e.g., linear interpolation processing, resolution increasing processing, etc.) on a received visible video and sends a visible video thus image-processed to the visible video/IR video superimposition processing unit 224.

The IR video processing unit 223 performs ordinary image processing (e.g., linear interpolation processing, resolution increasing processing, etc.) on a received IR video and sends an IR video thus image-processed to the visible video/IR video superimposition processing unit 224.

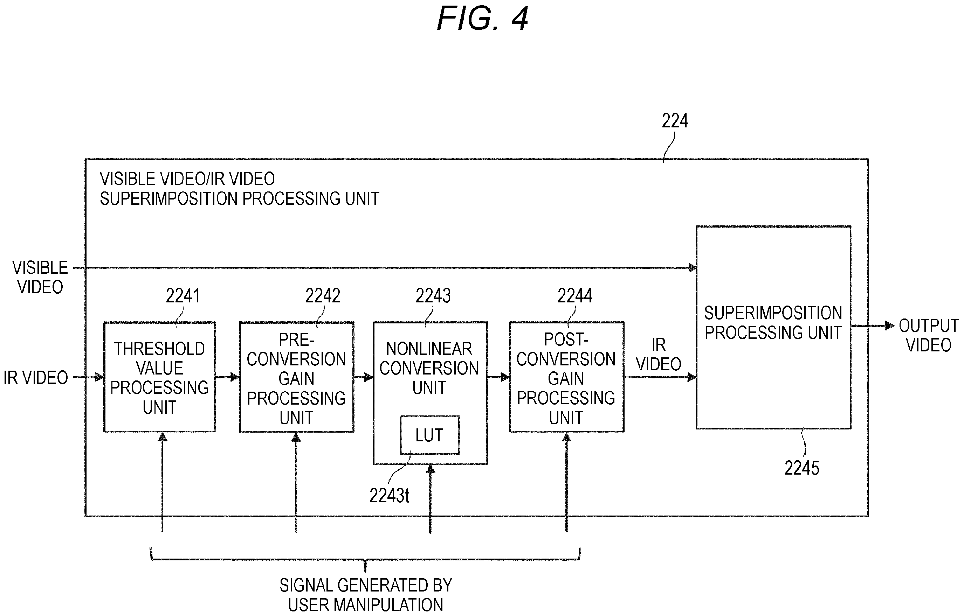

The visible video/IR video superimposition processing unit 224 performs various kinds of image processing on the IR video signal sent from the IR video processing unit 223, generates a superimposed video (superimposed image) by superimposing an image-processed IR video signal on the visible video signal sent from the visible video processing unit 222, and outputs the generated superimposed video (superimposed image) to the output unit 30. A detailed operation of the visible video/IR video superimposition processing unit 224 will be described later with reference to FIG. 4. Incidentally, a signal generated on the basis of a manipulation made on a manipulation unit (not shown) by a doctor or the like who looked at a superimposed video that was output to the output unit 30 (e.g., liquid crystal display) at the time of a microscope surgery or an endoscope surgery may be input to the visible video/IR video superimposition processing unit 224, and parameters (described later) of the image processing to be performed on an IR video signal may be changed according to that signal as appropriate.

The output unit 30 is a video display device configured using, for example, a liquid crystal display (LCD) or an organic EL (electroluminescence) display or a recording device for recording data of an output video (i.e., superimposed video (superimposed image)). The recording device is an HDD (hard disk drive) or an SSD (solid-state drive), for example.

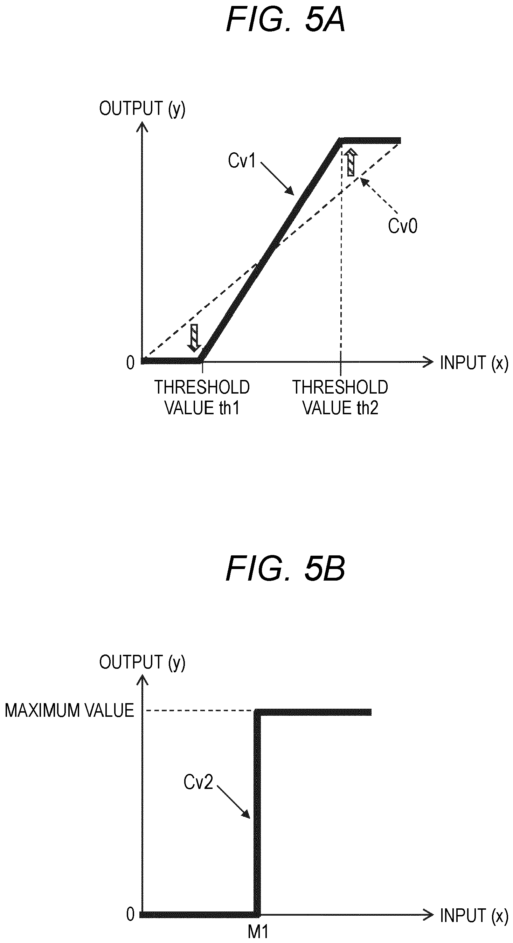

FIG. 4 is a block diagram showing an example detailed hardware configuration of the visible video/IR video superimposition processing unit 224. The visible video/IR video superimposition processing unit 224 is configured so as to include a threshold value processing unit 2241, a pre-conversion gain processing unit 2242, a nonlinear conversion unit 2243, a post-conversion gain processing unit 2244, and a superimposition processing unit 2245. Incidentally, in FIG. 4, the threshold value processing unit 2241 and the nonlinear conversion unit 2243 may be formed as the same circuit (e.g., nonlinear conversion unit 2243). This is because threshold value processing (described below) can be considered an example of nonlinear conversion processing and the nonlinear conversion unit 2243 can realize threshold value processing by implementing a characteristic (see FIG. 5A) to be used by the threshold value processing unit 2241 using a lookup table.

The threshold value processing unit 2241 performs intensity correction processing of decreasing the intensity of an input IR video signal (e.g., IR image brightness of each of pixels constituting the IR video or IR image brightness of each block consisting of k*k pixels (k is an integer that is a multiple of 2 and is larger than or equal to 2); this also applies to the following) if the intensity is lower than a first threshold value th1 and increasing the intensity of an input IR video signal if the intensity is higher than or equal to a second threshold value th2 (> (first threshold value th1)) (see FIG. 5A). The threshold value processing unit 2241 sends an IR video signal as subjected to the intensity correction processing to the pre-conversion gain processing unit 2242.

FIG. 5A is an explanatory diagram of the threshold processing. As described above, the threshold processing is the intensity correction processing of decreasing the intensity of an input IR video signal using parameters (e.g., two kinds of threshold values, that is, the first threshold value th1 and the second threshold value th2). In FIG. 5A, the horizontal axis (x axis) represents the intensity of an input IR video signal and the vertical axis (y axis) represents the intensity of an IR video signal that is output after the threshold value processing. A characteristic Cv1 represents a characteristic of the threshold value processing performed in the threshold value processing unit 2241. A characteristic Cv0 represents a characteristic of the threshold value processing unit 2241 in a case that the threshold value processing is not performed. In this characteristic, the input and the output are the same (y = x).

It is not rare that an IR video signal that is input to the threshold value processing unit 2241 contains noise components. If an IR video contains noise components, a white portion (in other words, a diseased portion that is an observation target portion fluorescing) in the IR video becomes dark partially and hence the details of the diseased portion are made unclear or a black portion (in other words, a background portion other than the diseased portion) in the IR video becomes a little whiter. As a result, the image quality of the IR video is lowered.

In view of the above, as shown in FIG. 5A, if the intensity of an input IR video signal is lower than the first threshold value th1, the threshold value processing unit 2241 decreases (in other words, suppresses) the intensity so that the output value becomes 0. That is, the threshold value processing unit 2241 corrects the intensity of each input pixel or block that is lower than the first threshold value th1 to 0 to thereby emphasize a black portion in the IR video.

As shown in FIG. 5A, if the intensity of an input IR video signal is higher than or equal to the second threshold value th2, the threshold value processing unit 2241 increases the output value to a prescribed maximum output value among expressible values. That is, the threshold value processing unit 2241 corrects the intensity of each input pixel or block that is higher than or equal to the second threshold value th2 to the maximum output value to thereby emphasize a white portion in the IR video.

As shown in FIG. 5A, if the intensity of an input IR video signal is higher than or equal to the first threshold value th1 and lower than the second threshold value th2, the threshold value processing unit 2241 corrects the intensity so that a dark portion in the IR video is made even darker and a whitish portion in the IR video is made even whiter. Thus, the gradation of the IR image can be made closer to black/white gradation, whereby a fluorescence portion can be made discernible when superimposed on a visible video. Incidentally, at least one of the first threshold value th1 and the second threshold value th2 may be changed on the basis of a signal that is input by a manipulation of the manipulation unit (not shown) made by a doctor or the like who looked at a superimposed video (superimposed image) displayed on the output unit 30 and a resulting value may be input to the threshold value processing unit 2241.

The pre-conversion gain processing unit 2242 holds a preset first gain value. The first gain value may be set on the basis of a signal that is input by a manipulation of the manipulation unit made by a doctor or the like who looked at a superimposed video displayed on the output unit 30. The pre-conversion gain processing unit 2242 receives an IR video signal objected by the intensity correction processing of the threshold value processing unit 2241 and amplifies it using the first gain value. The pre-conversion gain processing unit 2242 sends an amplified IR video signal to the nonlinear conversion unit 2243. As a result, in the camera device 20, amplification processing can be performed that allows the downstream nonlinear conversion unit 2243 to perform nonlinear conversion processing more easily and contribution to use of other kinds of image processing can be made, whereby the post-conversion gain processing unit 224 can be given versatility.

The nonlinear conversion unit 2243 is configured using a nonlinear processing circuit that holds a lookup table (LUT) 2243t and performs nonlinear processing using the lookup table 2243t. The nonlinear conversion unit 2243 performs nonlinear conversion processing on an IR video signal sent from the pre-conversion gain processing unit 2242 on the basis of groups of values written in the lookup table 2243t. For example, the nonlinear conversion processing is processing of converting the intensity of an IR video signal into a binary value or an N-ary value, that is, processing for representing an IR video signal in two or N gradation levels. N is an integer that is larger than or equal to 3. A kind of nonlinear conversion processing (e.g., binarization or conversion into an N-ary value) performed by the nonlinear conversion unit 2243 may be either set in advance or set on the basis of a signal that is input by a manipulation of the manipulation unit (not shown) made by a doctor or the like who looked at a superimposed video displayed on the output unit 30

The nonlinear conversion processing performed by the nonlinear conversion unit 2243 is not limited to the above-described processing performed using the lookup table 2243t, and may be nonlinear conversion processing that is performed by a polygonal approximation circuit that performs processing of connecting points by polygonal lines using a ROM (read-only memory) in which data of a nonlinear function (e.g., data of individual points of polygonal lines used for approximation) is stored. Usually, the lookup table is formed so as to have output values corresponding to respective values (e.g., 0 to 255 in the case of 8 bits) that can be taken by an input signal and consists of 256 data. Thus, the amount of data held by the lookup table tends to increase as the number of bits of values that can be taken by an input signal becomes larger. On the other hand, the polygonal approximation circuit can reduce the number of data to be held because the number of data to be held is only the number of points of polygonal lines.

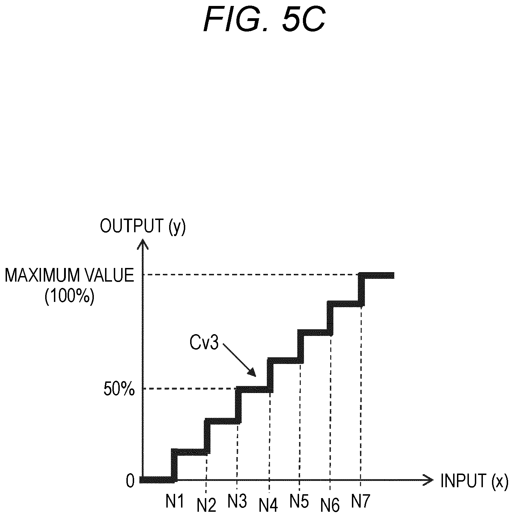

Incidentally, IR video (i.e., video taken through fluorescence emitted from a fluorescent chemical such as ICG when it is excited by IR excitation light) has a problem that it is difficult to see because it is lower in light intensity than ordinary visible light and when an IR video is superimposed on a visible video an IR portion (i.e., fluorescence portion) of the IR video is difficult to discriminate in the case where the difference in gradation from the visible video is large (e.g., in the case of 10 bits (i.e., 2¹⁰ = 1,024 gradation levels)). In view of this problem, in the first embodiment, as shown in FIGS. 5B or 5C, the nonlinear conversion unit 2243 performs nonlinear conversion processing (e.g., binarization or conversion into an N-ary value) on an input IR video signal and thereby generates an IR video signal that allows a fluorescence portion to be discriminated easily (in other words., less prone to be buried in an IRGB visible video portion) when superimposed on a visible video.

FIG. 5B is an explanatory diagram illustrating a first example (binarization) of the nonlinear conversion processing. FIG. 5C is an explanatory diagram illustrating a second example (conversion into an N-ary value) of the nonlinear conversion processing.

In FIG. 5B, the horizontal axis (x axis) represents the intensity of an IR video signal and the vertical axis (y axis) represents the intensity of an IR video signal that is output after the nonlinear conversion processing. As shown in FIG. 5B, the nonlinear conversion unit 2243 generates a conversion output "0" if the intensity of a pixel or a block (described above) of an input IR video signal is lower than M1 that is held by the lookup table 2243t. A characteristic Cv2 is an example characteristic of the nonlinear conversion processing performed by the nonlinear conversion unit 2243. Value groups of an input value and an output value for outputting an output value "0"if the input value is smaller than or equal to M1 and outputting a maximum output value (e.g., "1") if the input value is larger than M1 are written in the lookup table 2243t. With this measure, the camera device 20 can generate a superimposed video in which a fluorescence portion can be discriminated easily when superimposed on a visible video because an IR video signal can be expressed simply as 1 bit (black or white) depending on whether the input value (i.e., the intensity of each pixel or block of an input IR video signal) is smaller than or equal to M1.

In FIG. 5C, the horizontal axis (x axis) represents the intensity of an IR video signal and the vertical axis (y axis) represents the intensity of an IR video signal that is output after the nonlinear conversion processing. As shown in FIG. 5C, the nonlinear conversion unit 2243 converts an input IR video signal into an output value that is one of stepwise values according to a magnitude relationship between the intensity of a pixel or block (described above) of the input IR video signal and N1, N2, N3, N4, N5, N6, and N7 that are held in the lookup table 2243t. A characteristic Cv3 is an example characteristic of the nonlinear conversion processing performed by the nonlinear conversion unit 2243. Value groups of an input value and an output value for outputting an output value "0" if the input value is smaller than or equal to N1 and outputting a maximum output value (e.g., "8") if the input value is larger than N7 are written in the lookup table 2243t. A value that is 50% of the maximum value is assigned if the input value is larger than N3 and smaller than N4. That is, as shown in FIG. 5C, an output value is assigned as a result value of the nonlinear conversion processing represented by the characteristic Cv3 shown in FIG. 5C according to results of comparison between the intensity of each pixel or block of the input IR video signal and the seven values N1 to N7. With this measure, the camera device 20 can generate a superimposed video in which a fluorescence portion can be discriminated easily when superimposed on a visible video because an IR video signal can be expressed finely in 8-bit grayscale according to magnitude relationships between the input value (i.e., the intensity of each pixel or block of an input IR video signal) and the seven values N1 to N7.

The post-conversion gain processing unit 2244 holds a preset second gain value. The second gain value may be set on the basis of a signal that is input by a manipulation of the manipulation unit made by a doctor or the like who looked at a superimposed video displayed on the output unit 30. The post-conversion gain processing unit 2244 receives an IR video signal obtained by the nonlinear conversion processing of the nonlinear conversion unit 2243 and amplifies it using the second gain value. The post-conversion gain processing unit 2244 sends an amplified IR video signal to the superimposition processing unit 2245. As a result, in the camera device 20, since the color is made deeper by the amplification of the IR video signal as subjected to the nonlinear conversion processing, amplification processing can be performed that allows a fluorescence portion of an IR video to be discriminated more easily when the IR video is superimposed on a visible video and contribution to use of other kinds of image processing can be made, whereby the visible video/IR video superimposition processing unit 224 can be given versatility.

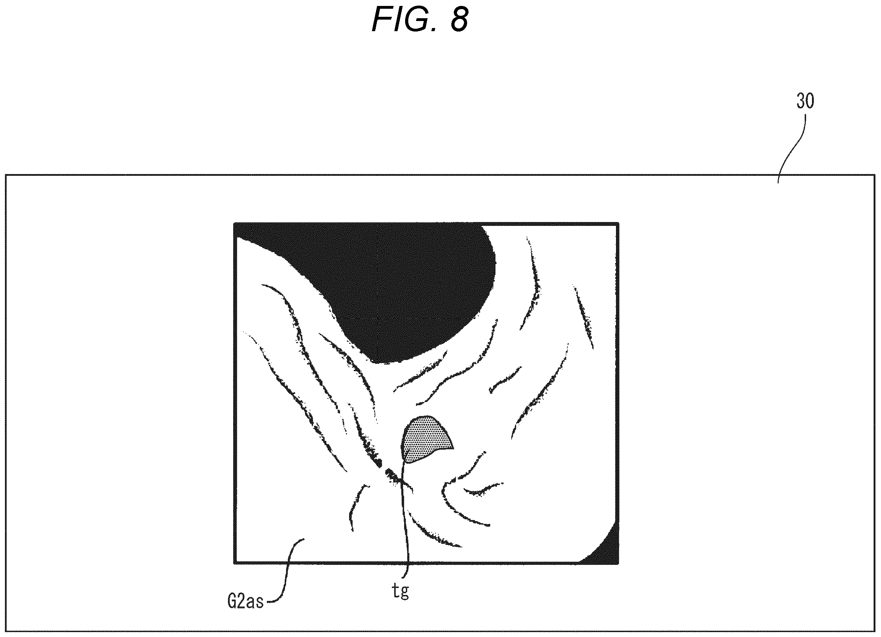

The superimposition processing unit 2245 receives a visible video signal sent from the visible video processing unit 222 and an IR video signal sent from the post-conversion gain processing unit 2244 and generates a superimposed video (superimposed image) in which the IR video signal is superimposed on the visible video signal (see FIG. 8). The superimposition processing unit 2245 outputs a superimposed video signal to the output unit 30 (e.g., monitor) as an output video signal.

The superimposition processing unit 2245 can generate a superimposed video by performing example superimposition processing, that is, superimposing an IR video portion on a visible video and coloring the IR video potion in green by adding, to RGB information (pixel values) of each block of k*k pixels of the visible video (visible image), G (green) information (pixel values) of the same block of the corresponding IR video. The superimposition processing performed in the superimposition processing unit 2245 is not limited to the above example processing.

FIG. 8 shows an example superimposed video G2as in which an IR video is superimposed on a visible video. The superimposed video G2as is colored in, for example, green to make a state of a white portion (i.e., a diseased portion tg that is a non-background portion and in which a fluorescent chemical such as ICG is fluorescing) of the IR video easier to recognize when the IR video is superimposed on a visible video. A doctor or the like can visually recognize a light emission distribution etc. of the fluorescent chemical administered to the inside of the subject body 40 and a state of the diseased portion tg by looking at the superimposed video G2as that has been output to and is displayed on the output unit 30 (e.g., monitor).

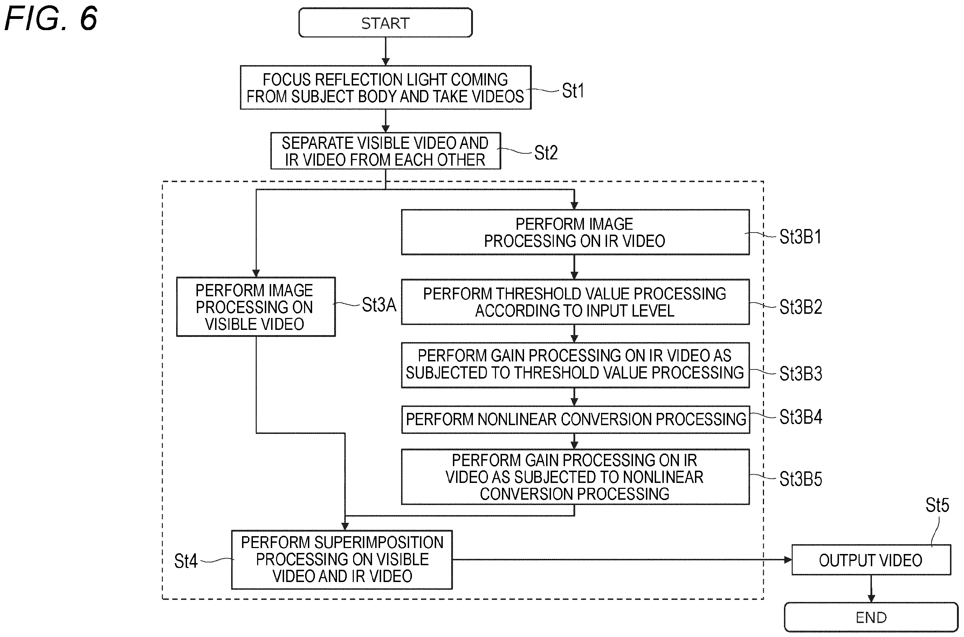

Next, an example operation procedure of the camera device 20 according to the first embodiment will be described with reference to FIG. 6. FIG. 6 is a flowchart showing an example operation procedure of the camera device 20 according to the first embodiment. The steps enclosed by a broken line in FIG. 6 is executed for each visible image frame of a visible video or for each IR image frame of an IR video.

Referring to FIG. 6, in the camera device 20, the camera head 21 focuses reflection light coming from a subject body 40 (i.e., visible light and fluorescence reflected by the subject body 40) on the imaging unit 24 and the imaging unit 24 takes a visible video and an IR video (St1) . Incidentally, in the first embodiment, it is preferable that transmission of IR excitation light for exciting a fluorescent chemical that was administered to the inside of the subject body 40 in advance (described above) be prevented by, for example, a band cut filter disposed between the objective lens 11 and each of the zoom optical systems 101L and 101R. This suppresses entrance of reflection light of the IR excitation light into the camera head 21 of the camera device 20, whereby the image quality of a signal of a fluorescence observation video is made high.

In the camera device 20, an observation video signal transmitted from the camera head 21 by the signal cable 25 is separated into a visible video signal and an IR video signal which are sent to the visible video processing unit 222 and the IR video processing unit 223, respectively (St2).

The camera device 20 performs ordinary image processing (e.g., linear interpolation processing, resolution increasing processing, etc.) for each visible image frame of the visible video (St3A).

The camera device 20 performs ordinary image processing (e.g., linear interpolation processing, resolution increasing processing, etc.) for each IR image frame of the IR video (St3B1). The camera device 20 performs, for each IR image frame obtained by the image processing at step St3B1, intensity correction processing of decreasing its intensity if the frame intensity (e.g., the IR image brightness of each of pixels constituting the IR video or the IR image brightness of each block consisting of k*k pixels) is lower than the first threshold value th1 and increasing its intensity if the frame intensity is higher than or equal to the second threshold value th2 (> (first threshold value th1)) (St3B2).

The camera device 20 amplifies, for each frame that was subjected to the intensity correction processing (threshold value processing) at step St3B2, an IR video signal as subjected to the intensity correction processing using the first gain value (St3B3). The camera device 20 performs nonlinear conversion processing for each IR image frame of an IR video signal as amplified at step St3B3 on the basis of the value groups written to the lookup table 2243t (St3B4). The camera device 20 amplifies an IR video signal as subjected to the nonlinear conversion processing using the second gain value for each IR image frame of the IR video as subjected to the nonlinear conversion processing at step St3B4 (St3B5).

Using a visible video signal as subjected to the image processing at step St3A and an IR video signal as subjected to the amplification at step St3B5, the camera device 20 generates a superimposed video (superimposed image) in which the IR video signal is superimposed on the visible video signal (St4). The camera device 20 outputs the superimposed video signal generated at step St4 to the output unit 30 as an output video (St5).

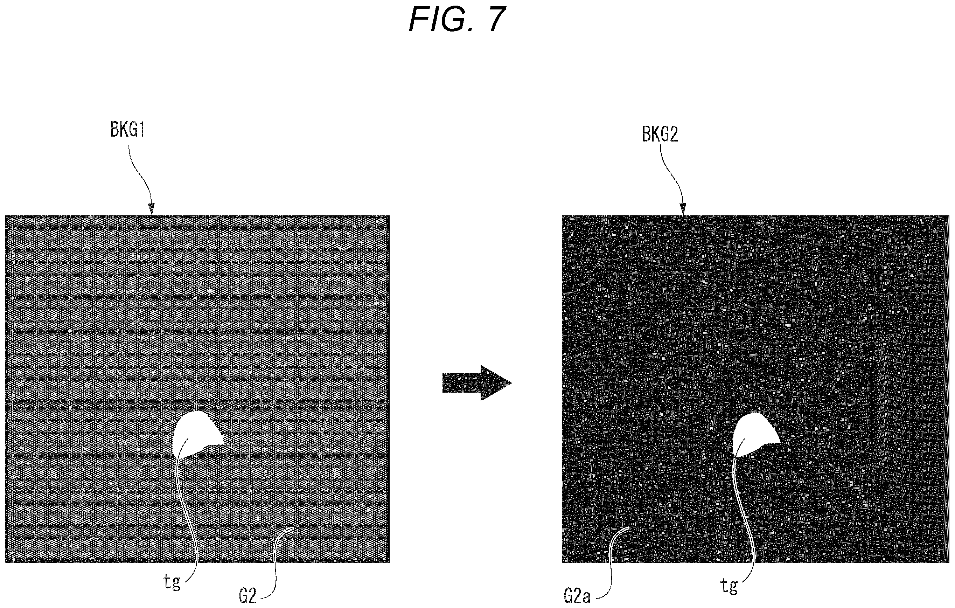

FIG. 7 is example schematic views showing how an IR video is varied by the threshold processing and the nonlinear conversion processing. In FIG. 7, an IR video G2 is one that has not been subjected to the threshold processing and the nonlinear conversion processing yet. In the IR video G2 shown in FIG. 7, strong noise components exist in a wide portion BKG1 other than a diseased portion tg (i.e., the entire video excluding the diseased portion tg and a portion around it) and the color of the portion BKG1 is close to black to gray. Thus, when looking at, on the output unit 30 (e.g., monitor), a superimposed video in which the IR video G2 is superimposed on a visible video, a doctor or the like has difficulty recognizing the details of a boundary portion of the diseased portion tg and may make an erroneous judgment at the time of a surgery or an examination as to what extent the diseased portion extends in the IR video G2.

On the other hand, an IR video G2a is one that has been subjected to the threshold processing and the nonlinear conversion processing. The strong noise components that exist in the portion BKG1 of the IR video G2 other than the diseased portion tg are eliminated and the IR video G2a shown in FIG. 7 is clear in image quality, and the color of a portion BKG2 other than the diseased portion tg is very close to black. As such, the IR video G2a is improved in image quality from the IR video G2. Thus, when looking at, on the output unit 30 (e.g., monitor), a superimposed video in which the IR video G2a is superimposed on a visible video, a doctor or the like can easily recognize visually the details of an outline of the diseased portion tg and the inside of it and hence can perform a surgery or an examination smoothly.

As described above, the camera device 20 according to the first embodiment performs each of imaging on the basis of visible light shining on the medical optical device from an observation target part (e.g., a diseased portion that is a target part of a surgery) of a subject body (e.g., patient) to which a fluorescent chemical such as ICG (indocyanine green) was administered in advance and imaging on the basis of fluorescence shining on the medical optical device from the observation target part. For example, the medical optical device is a surgical microscope or a surgical endoscope. The camera device performs image processing including at least nonlinear conversion processing on a fluorescence image obtained by the imaging on the basis of fluorescence and generates a superimposed image to be output to the output unit by superimposing a fluorescence image as subjected to the image processing on a visible image obtained by the imaging on the basis of visible light.

Having the above configuration, the camera device 20 can suppress lowering of the image quality of the fluorescence image because it can determine a hue of the fluorescence image using the number of bits that is smaller than the number of bits that determines a hue of the visible image by performing nonlinear conversion processing on a fluorescence image carried by fluorescence emitted by the fluorescent chemical such as ICG. Furthermore, since the camera device 20 performs the nonlinear conversion processing so as to make a fluorescence image a clear black-and-white image, a fluorescence portion of the fluorescence image is easy to see when it is superimposed on an ordinary visible image. As such, the camera device 20 can assist output of a video taken that allows a user such as a doctor to check a clear state of a target part of a subject body.

The camera device 20 amplifies the intensity of a fluorescence image received from the camera head 21 and then performs the nonlinear conversion processing on the amplified fluorescence image. With this measure, the camera device 20 can perform amplification processing for allowing the downstream nonlinear conversion unit 2243 to perform the nonlinear conversion processing more easily and contribution to use of other kinds of image processing can be made. The visible video/IR video superimposition processing unit 224 can thus be given versatility.

The camera device 20 amplifies the intensity of the fluorescence image as subjected to the nonlinear conversion processing. With this measure, in the camera device 20, since the color is made deeper by amplification of an IR video signal as subjected to the nonlinear conversion processing, amplification processing can be performed that allows a fluorescence portion of an IR video to be discriminated more easily when the IR video in the downstream superimposition processing unit 2245 is superimposed on a visible video and contribution to use of other kinds of image processing can be made. The visible video/IR video superimposition processing unit 224 can thus be given versatility.

The amplification factor of the amplification performed by the pre-conversion gain processing unit 2242 or the post-conversion gain processing unit 2244 can be varied by manipulation of a user such as a doctor. With this measure, the doctor or the like can adjust, properly, the visibility of a superimposed image that is output to the output unit 30 (in other words, a diseased portion tg of a subject body 40) and hence make a correct judgment in a microscope surgery or an endoscope surgery.

The camera device 20 performs intensity amplification processing that lowers the intensity of a fluorescence image if the intensity is lower than the first threshold value th1 and increases the intensity of a fluorescence image if the intensity is higher than or equal to the second threshold value th2 that is larger than the first threshold value th1, and performs the nonlinear conversion processing on a fluorescence image as subjected to the intensity amplification processing. With this measure, since the camera device 20 can eliminate the influence of noise components effectively by the intensity correction processing even if an IR image taken includes the noise components, the gradation of the IR image can be made closer to black/white two-level gradation, whereby a fluorescence portion can be made discernible when superimposed on a visible video.

In the intensity correction processing (threshold processing), the first threshold value th1 and the second threshold value th2 can be varied by a manipulation user such as a doctor. With this measure, the doctor or the like can judge whether the boundary of a diseased portion tg of a subject body 40 in a superimposed image that is output to the output unit 30 is hard to see being buried in a visible video around it and, if the diseased portion tg is hard to see, can adjust the first threshold value th1 and the second threshold value th2 as appropriate. This allows the doctor or the like to make a correct judgment in a microscope surgery or an endoscope surgery.

Furthermore, the camera device 20 has the lookup table 2243t having value groups that can be changed by a manipulation of a user such as a doctor and performs the nonlinear conversion processing using the lookup table 2243t. With this measure, the camera device 20 can generate a superimposed video that enables easy discrimination of a fluorescence portion when it is superimposed on a visible video because an IR video signal can be expressed by one bit (black or white) simply depending on whether the input value (i.e., the intensity of each pixel or block of an input IR video signal) is smaller than or equal to M1. Alternatively, the camera device 20 can generate a superimposed video that enables easy discrimination a fluorescence portion when it is superimposed on a visible video because an IR video signal can be expressed finely in 8-bit grayscale according to magnitude relationships between the input value (i.e., the intensity of each pixel or block of an input IR video signal) and the seven values N1 to N7. In these manners, since a doctor or the like can select nonlinear conversion processing to be employed in the nonlinear conversion unit 2243 by, for example, switching among lookup tables 2243t as appropriate to check the visibility of a superimposed image (in other words, a diseased portion tg of a subject body 40) that is output to the output unit 30, the camera device 20 can perform proper nonlinear conversion processing and the doctor or the like can see a superimposed video having good image quality.

### (Embodiment 2)

A camera device according to a second embodiment is equipped with, in addition to the configuration of the above-described camera device according to the first embodiment, at least one selection unit which selects at least one of a visible image, a fluorescence image as subjected to image processing, and a superimposed image and outputs the at least one selected image to at least one corresponding output unit.

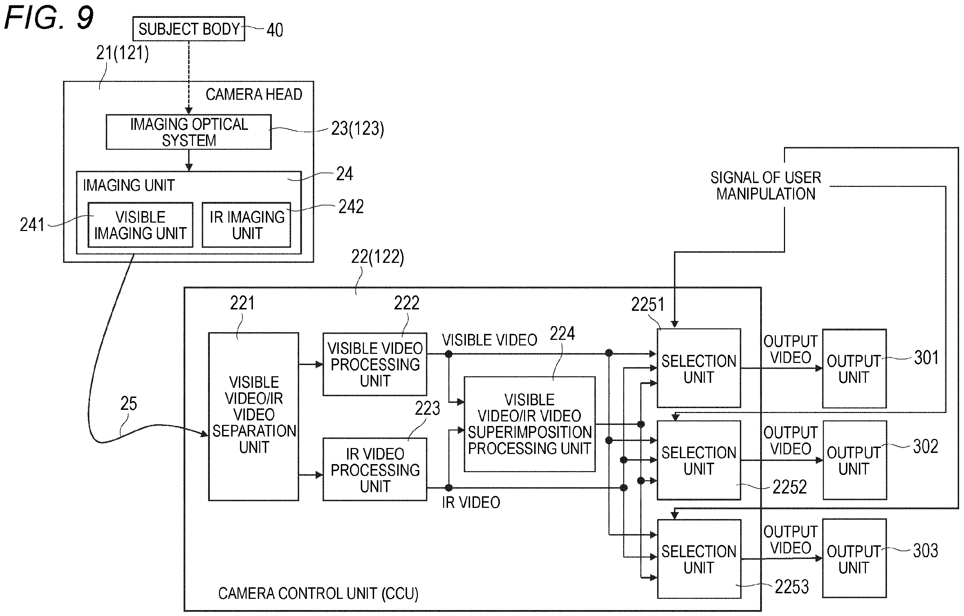

FIG. 9 is a block diagram showing an example hardware configuration of the camera device 20 according to the second embodiment. In the description to be made with reference to FIG. 9, constituent elements having the same ones in the description that was made with reference to FIG. 3 will be given the same symbols as the latter and omitted or described briefly. Only different constituent elements will be described.

The camera device 20 shown in FIG. 9 is configured so as to include a camera head 21 or 121 and a CCU 22 or 122. The CCU 22 or 122 is configured so as to include a visible video/IR video separation unit 221, a visible video processing unit 222, an IR video processing unit 223, a visible video/IR video superimposition processing unit 224, and selection units 2251, 2252, and 2253.

A visible video signal as subjected to image processing by the visible video processing unit 222, an IR video signal as subjected to image processing by the IR video processing unit 223, and a superimposed video signal generated by the visible video/IR video superimposition processing unit 224 are input to the selection unit 2251. The selection unit 2251 selects at least one of the visible video, the IR video, and the superimposed video according to a signal that is input by a manipulation of a user such as a doctor and outputs the selected video to an output unit 301.

The visible video signal as subjected to the image processing by the visible video processing unit 222, the IR video signal as subjected to the image processing by the IR video processing unit 223, and the superimposed video signal generated by the visible video/IR video superimposition processing unit 224 are input to the selection unit 2252. The selection unit 2252 selects at least one of the visible video, the IR video, and the superimposed video according to the signal that is input by the manipulation of the user such as a doctor and outputs the selected video to an output unit 302.

The visible video signal as subjected to the image processing by the visible video processing unit 222, the IR video signal as subjected to the image processing by the IR video processing unit 223, and the superimposed video signal generated by the visible video/IR video superimposition processing unit 224 are input to the selection unit 2253. The selection unit 2253 selects at least one of the visible video, the IR video, and the superimposed video according to the signal that is input by the manipulation of the user such as a doctor and outputs the selected video to an output unit 303.

Each of the output units 301, 302, and 303 is a video display device configured using, for example, a liquid crystal display (LCD) or an organic EL (electroluminescence) display or a recording device for recording data of an output video (i.e., superimposed video (superimposed image)). The recording device is an HDD (hard disk drive) or an SSD (solid-state drive), for example.

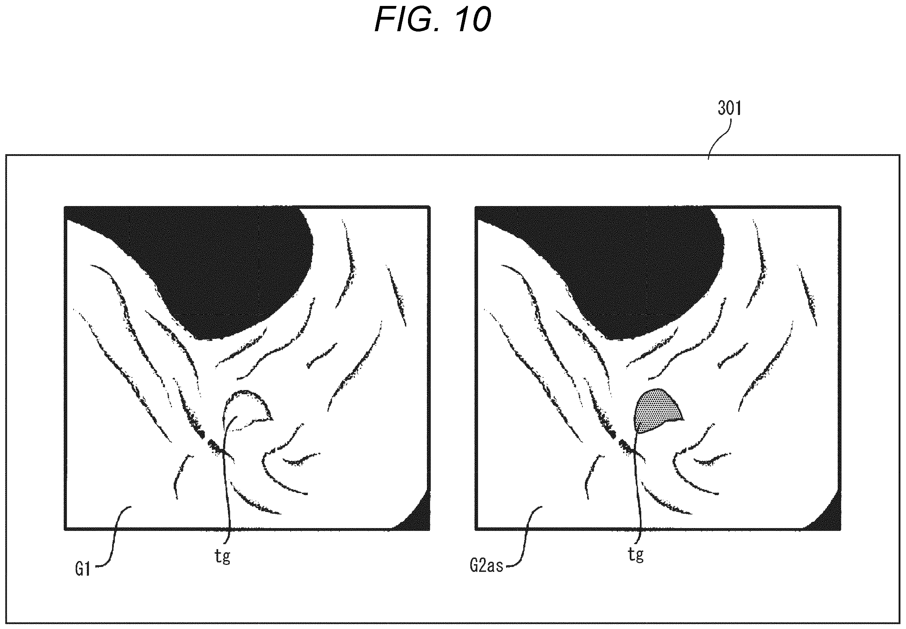

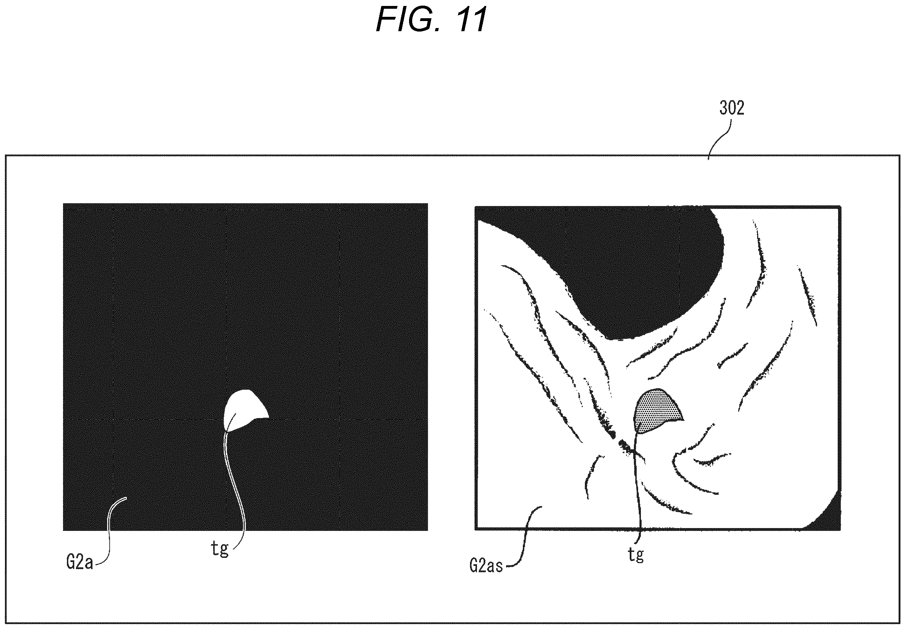

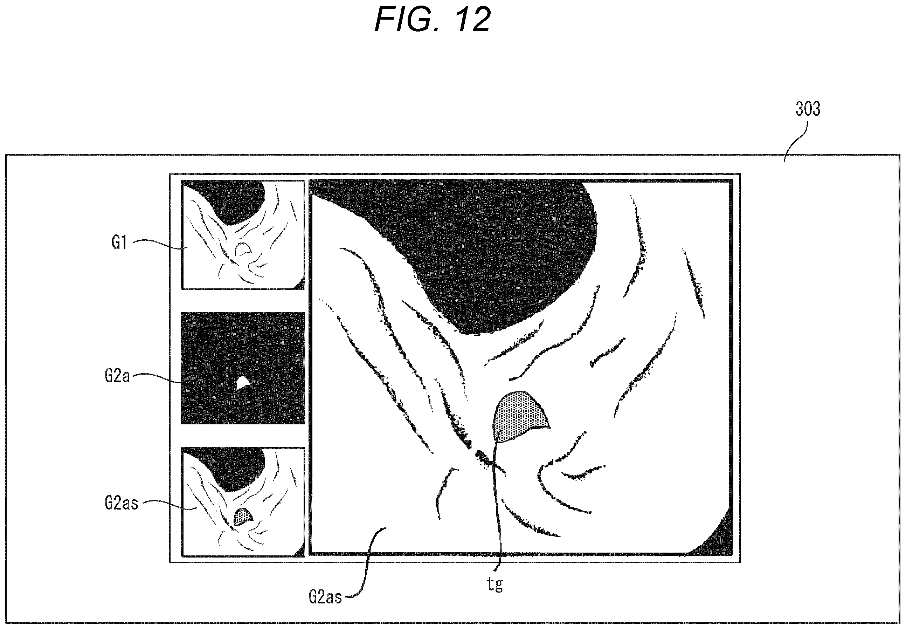

FIG. 10 is views showing display examples for comparison between a visible video G1 and a superimposed video G2as. FIG. 11 is views showing display examples for comparison between an IR video G2a and the superimposed video G2as. FIG. 12 is views showing display examples for comparison between the visible video G1, the IR video G2a, and the superimposed video G2as.

As shown in FIG. 10, the camera device 20 according to the second embodiment can select, for example, the visible video G1 and the superimposed video G2as by the selection unit 2251 and output them to the output unit 301. That is, in contrast to the camera device 20 according to the first embodiment which outputs the superimposed video G2as to the output unit 30, the camera device 20 according to the second embodiment can select plural kinds of videos and display them on the same screen in such a manner that they are compared with each other. This allows a doctor or the like to check, in a simple manner, whether the degree of superimposition of a fluorescence portion (i.e., a video of a diseased portion tg) in the superimposed video G2as is proper while comparing the superimposed video G2as and the visible video G1 on the same output unit 301 and to, if necessary, adjust the degree of superimposition by changing various parameters in the manner described in the first embodiment.

As shown in FIG. 11, the camera device 20 according to the second embodiment can select, for example, the IR video G2a and the superimposed video G2as by the selection unit 2252 and output them to the output unit 302. That is, in contrast to the camera device 20 according to the first embodiment which outputs the superimposed video G2as to the output unit 30, the camera device 20 according to the second embodiment can select plural kinds of videos and display them on the same screen in such a manner that they are compared with each other. This allows a doctor or the like to check, in a simple manner, whether noise components are suppressed properly in the IR video G2a and whether the intensity of a fluorescence portion (i.e., a video of a diseased portion tg) is corrected properly so that the fluorescence portion can be discriminated in the superimposed video G2as while comparing the superimposed video G2as and the IR video G2a on the same output unit 302 and to, if necessary, correct the image quality of the IR video G2a by changing various parameters in the manner described in the first embodiment.

As shown in FIG. 12, the camera device 20 according to the second embodiment can select, for example, the visible image G1, the IR video G2a, and the superimposed video G2as by the selection unit 2253 and output them to the output unit 303. That is, in contrast to the camera device 20 according to the first embodiment which outputs the superimposed video G2as to the output unit 30, the camera device 20 according to the second embodiment can select plural kinds of videos and display them on the same screen in such a manner that they are compared with each other. Although FIG. 12 shows a display mode in which the screen is divided into four parts and the superimposed video G2as is displayed so as to have a widest display area, the invention is not limited to this example. This allows a doctor or the like to check, in a simple manner, whether the degree of superimposition of a fluorescence portion (i.e., a video of a diseased portion tg) in the superimposed video G2as is proper and while comparing the visible video G1, the IR video G2a, and the superimposed video G2as on the same output unit 303. Furthermore, the doctor or the like can check, in a simple manner, whether noise components are suppressed properly in the IR video G2a and whether the intensity of a fluorescence portion (i.e., a video of a diseased portion tg) is corrected properly so that the fluorescence portion can be discriminated in the superimposed video G2as. Thus, the doctor or the like can correct the image quality of the IR video G2a by changing various parameters in the manner described in the first embodiment.

Although FIGS. 10, 11, and 12 show the examples in which plural videos are selected by each of the selection units 2251, 2252, and 2253 and displayed on each of the output units 301, 302, and 303, each of the selection units 2251, 2252, and 2253 may select only one of the visible video G1, the IR video G2a, and the superimposed video G2as and output it to the corresponding output unit 301, 302, or 303. In this case, the camera device 20 etc. can output display target videos by making full use of the display area of each of the output units 301, 302, and 303 and hence allow a doctor or the like to recognize the details of each output video.

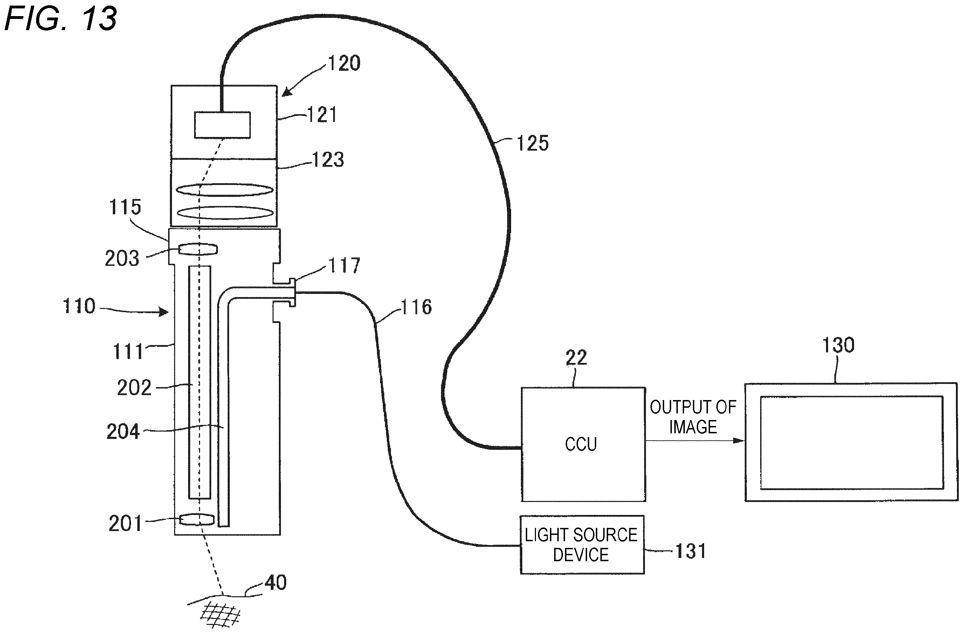

FIG. 13 is a system configuration diagram showing an example configuration in which a medical camera system including a camera device 20 according to the first or second embodiment is applied to a surgical endoscope system. The surgical endoscope system is equipped with a surgical endoscope 110, a camera device 120, an output unit 130 (e.g., a display device such as a monitor), and a light source device 131. The camera device 120 is the same as the camera device 20 shown in FIGS. 1-4 and is configured so as to have a camera head 121 and a CCU 122.

The surgical endoscope 110 is configured so as to have, in a long insertion unit 111, an objective lens 201L, a relay lens 202L, and an image forming lens 203L. The surgical endoscope 110 is equipped with a camera mounting unit 115 which is provided on the proximal side of an observation optical system, a light source attaching portion 117, and a light guide 204 for guiding illumination light from the light source attaching portion 117 to a tip portion of the insertion unit 111. The camera device 120 can acquire an observation video by attaching an imaging optical system 123 of the camera head 121 to the camera mounting unit 115 and performs imaging. A light guide cable 116 is connected to the light source attaching portion 117 and the light source device 131 is connected by the light guide cable 116.

The camera head 121 and the CCU 122 are connected to each other by a signal cable 125, and a video signal of a subject body 40 taken by the camera head 121 is transmitted to the CCU 122 by the signal cable 125. The output unit 130 (e.g., a display device such as a monitor) is connected to an output terminal of the CCU 122. Either two (left and right) output videos (output video-1 and output video-2) for 3D display or a 2D observation video (observation image) may be output to the output unit 130. Either a 3D video having 2K pixels or a 2D observation video (observation image) may be output to the output unit 130 as an observation video of a target part.

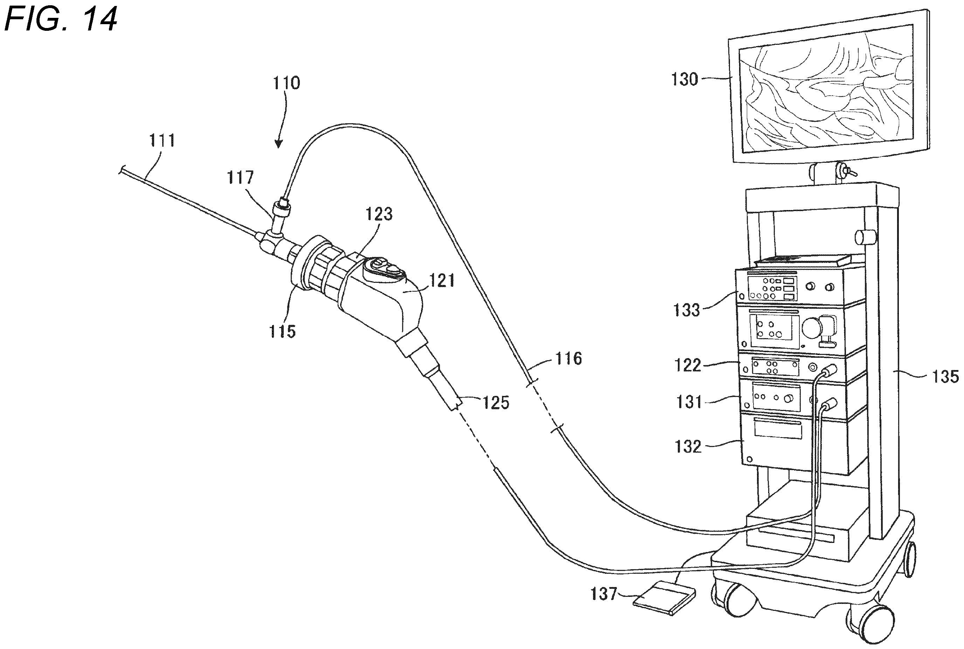

FIG. 14 shows an example appearance of the surgical endoscope system. In the surgical endoscope 110, the camera mounting unit 115 is provided on the proximal side of the insertion unit 111 and the imaging optical system 123 of the camera head 121 is attached to the camera mounting unit 115. The light source attaching portion 117 is provided on the proximal side of the insertion unit 111 and the light guide cable 116 is connected to the light source attaching portion 117. The camera head 121 is provided with manipulation switches, whereby a user can make a manipulation on an observation video taken (freezing, release manipulation, image scanning, or the like) by his or her hands. The surgical endoscope system is equipped with a recorder 132 for recording an observation image taken by the camera device 120, a manipulation unit 133 for manipulating the surgical endoscope system, and a foot switch 137 that allows an observer to make a manipulation input by his or her foot. The manipulation unit 133, the CCU 122, the light source device 131, and the recorder 132 are housed in a control unit chassis 135. The output unit 130 is disposed at the top of the control unit chassis 135.

As such, like the configuration of the above-described medical camera system, the configuration of the surgical endoscope system shown in FIGS. 13 and 14 makes it possible to output a superimposed video that is acquired through the surgical endoscope 110 and enables a check of a clear state of an observation target part.

Although the various embodiments have been described above with reference to the accompanying drawings, it goes without saying that the disclosure is not limited to that example. It is apparent that those skilled in the art could conceive various changes, modifications, replacements, additions, deletions, or equivalents within the confines of the claims, and they are construed as being included in the technical scope of the disclosure. And constituent elements of the above-described various embodiments may be combined in a desired manner without departing from the spirit and scope of the invention.

The present application is based on Japanese Patent Application No. 2018-105397 filed on May 31, 2018, the disclosure of which is incorporated herein by reference.

### INDUSTRIAL APPLICABILITY

The present disclosure is useful when applied to camera devices, image processing methods, and camera systems that suppress lowering of the image quality of a fluorescence image and make a fluorescence portion in a fluorescence image easier to see when the fluorescence image is superimposed on an ordinary visible image and thereby assist output of a video taken that allows a user such as a doctor to check a clear state of a target part of a subject body.

### DESCRIPTION OF SYMBOLS

- 10:: Surgical microscope
- 20, 120:: Camera device
- 21, 121:: Camera head
- 22, 122:: CCU (camera control unit)
- 23, 123:: Imaging optical system
- 24:: Imaging unit
- 25, 125:: Signal cable
- 30, 130, 301, 302, 303:: Output unit
- 40:: Subject body
- 110:: Surgical endoscope
- 221:: Visible video/IR video separation unit
- 222:: Visible video processing unit
- 223:: IR video processing unit
- 224:: Visible video/IR video superimposition processing unit
- 2241:: Threshold value processing unit
- 2242:: Pre-conversion gain processing unit
- 2243:: Nonlinear conversion unit
- 2244:: Post-conversion gain processing unit
- 2245:: Superimposition processing unit
- 2251, 2252, 2253:: Selection unit

## Claims

1. A camera device comprising:
a camera head which performs both of imaging on the basis of visible light shining on a medical optical device from a target part of a subject body to which a fluorescent chemical was administered in advance and imaging on the basis of fluorescence shining on the medical optical device from the target part; and
an image processing unit which performs nonlinear conversion processing on an amplified fluorescence image after the intensity of a fluorescence image that is input from the camera head is amplified and a black portion and a white portion of the fluorescence image are emphasized, and generates a superimposed image to be output to an output unit by superimposing a fluorescence image as subjected to the nonlinear conversion processing on a visible image obtained by imaging on the basis of visible light.

2. The camera device according to claim 1,
wherein the image processing unit lowers the intensity of the fluorescence image that is input from the camera head if the intensity of the fluoresces image is less than a first threshold value and increases the intensity of the fluorescence image if the intensity is higher than or equal to a second threshold value that is larger than the first threshold value.

3. The camera device according to claim 1 or 2,
wherein the image processing unit amplifies the intensity of the fluorescence image as subjected to the nonlinear conversion processing.

4. The camera device according to any one of claims 1 to 3,
wherein an amplification factor of the amplification is varied by a user manipulation.

5. The camera device according to any one of claims 2 to 4,
wherein the first threshold value and the second threshold value is varied by a user manipulation.

6. The camera device according to any one of claims 1 to 5,
wherein the image processing unit has a lookup table having value groups that is able to be changed by a user manipulation and performs the nonlinear conversion processing based on the lookup table.

7. The camera device according to any one of claims 1 to 6, further comprising at least one selection unit which selects at least one of the visible image, a fluorescence image as subjected to image processing, and the superimposed image and which outputs the at least one selected image to at least one corresponding output unit.

8. An image processing method of a camera device including a camera head and an image processing unit, the image processing method comprising the steps of:
causing the camera head to perform each of imaging on the basis of visible light shining on a medical optical device from a target part of a subject body to which a fluorescent chemical was administered in advance and imaging on the basis of fluorescence shining on the medical optical device from the target part; and
causing the image processing unit to perform nonlinear conversion processing on an amplified fluorescence image after the intensity of a fluorescence image that is input from the camera head is amplified and a black portion and a white portion of the fluorescence image are emphasized, and generates a superimposed image to be output to an output unit by superimposing a fluorescence image as subjected to the nonlinear conversion processing on a visible image obtained by the imaging on the basis of visible light.

9. A camera system comprising:
a camera device; and
an output unit,
wherein the camera device
performs each of imaging on the basis of visible light shining on a medical optical device from a target part of a subject body to which a fluorescent chemical was administered in advance and imaging on the basis of fluorescence shining on the medical optical device from the target part; and
performs nonlinear conversion processing on an amplified fluorescence image after the intensity of a fluorescence image that is obtained by the imaging on the basis of the fluorescent is amplified and a black portion and a white portion of the fluorescence image are emphasized, and generates a superimposed image to be output to an output unit by superimposing a fluorescence image as subjected to the nonlinear conversion processing on a visible image obtained by the imaging on the basis of visible light; and
the output unit outputs the superimposed image generated by the camera device.
